# EUROPEAN PATENT SPECIFICATION

(11) **EP 1 378 229 B1**
(45) Date of publication and mention of the grant of the patent: **12.10.2005**
(21) Application number: 03012999.3
(22) Date of filing: 10.06.2003
(51) Int. Cl.: A61K 7/13

(54) **Composition for the dyeing of human hair**
Haarfärbemittel
Composition de teinture des cheveux

(30) Priority: 03.07.2002 DE 10229743
(43) Date of publication of application: 07.01.2004
(73) Proprietor: KPSS-Kao Professional Salon Services GmbH, 64297 Darmstadt (DE)
(72) Inventor: Lorenz, Heribert, 64401 Gross-Bieberau (DE); Kaffenberger, Klaus, 64665 Alsbach-Hähnlein (DE)

(56) References cited:
- DE-U- 20 017 642

## Description

The present invention concerns a composition for the dyeing of human hair on the basis of an oxidation dyestuff precursor system reacting with peroxide which provides long-lasting, intensive colors either used as such, or which can be used to obtain further shades in combination with additional developing and/or coupling agents and which does not damage the hair even upon repeated application within short intervals.

The developing substances still most frequently used in hair dyeing compositions are 1,4-diaminobenzene (p-phenylenediamine) and 1-methyl-2,5-diaminobenzene (p-toluylenediamine). Although incorporation of these substances largely fulfills the user's color wishes, there are still shades that cannot be completely achieved by the use thereof.

Proposals have also been made to close this gap by the use of alternative developing substances. To a limited degree this is possible with the use of tetraaminopyrimidine or 2-(2,5-diaminophenyl)ethanol (see EP-B 400 330); however, it is then necessary to accept reduced color intensity in other shades.
A further satisfactory solution of this problem is disclosed in EP-A 615 743, with the use of 2-(2'-hydroxyethyl amino)-5-aminotoluene or the water-soluble salts thereof, as a component of oxidation hair dye compositions.

DE 200 17 642 U discloses hair dyeing compositions containing 2-chloro-p-aminophenol and o-aminophenol. Hair dyeing compositions are disclosed in DE 100 51 034 A1 comprising 2-chloro-p-aminophenol and m-aminophenol. Both documents are silent on a composition according to claim 1 of the current invention.

However, to the present it has not been possible to achieve strong colorations in the range of orange-red by this means.

The invention starts from the task of counteracting this deficiency and providing an oxidation dyestuff composition which achieves intensive, glossy colorations, especially in the range of orange and red, and which leaves the hair without damage even upon repeated application within short periods of time.

This task is solved when such a hair dyeing composition comprises an oxidation dyestuff system reacting with peroxide which is selected from each group a) 3-chloro-p-aminophenol and b) 2-methyl-5-aminophenol, 2-methyl-5-hydroxyethyl aminophenol, 2-methyl-5-γ-hydroxypropyl aminophenol, 2-methyl-5-methyl aminophenol, 2-methyl-5-ethyl aminophenol, 2-methoxy-5-aminophenol, 2-methyl-4-methoxy-5-aminophenol and/or 2-methyl-4-methoxy-5-hydroxyethyl aminophenol and c) o-aminophenol and/or 4-chloro-2-aminophenol.

After oxidation with peroxide, use of these compositions on the basis of a customary carrier provides very expressive, intensive, long-lasting hair colorations, especially in the range of orange and red, which can be varied to achieve further shades by the addition of the respective further developing and coupling substances.

In addition to the named developing and coupling substances it is also possible to incorporate further substances of this type.
Further suitable coupling substances are, for example, 1-methoxy-2-amino-4-(β-hydroxyethyl amino)benzene, 2-amino-3-hydroxypyridine, 3-amino-2-methylamino-6-methoxypyridine, resorcinol, 2-methyl resorcinol, 4-chlororesorcinol, 1,3-diamino benzene, 1,6-dihydroxynaphthaline, 1,7-dihydroxy-naphthaline, p-phenylenediamine, p-toluylenediamine, 2,6-dimethyl-p-phenylenediamine, 2-hydroxymethyl-p-phenylenediamine, 2-hydroxyethyl-p-phenylenediamine, 2-n-propyl-p-phenylenediamine, N,N-bis(β-hydroxyethyl)-p-phenylenediamine, N-methoxyethyl-p-phenylenediamine and/or 5-chloro-2-hydroxyethyl-p-phenylenediamine or the water-soluble salts thereof.
The total concentration of the developing substances is customarily from 0.05 % to 5 %, preferably 0.1 % to 4 %, in particular 0.25 % to 0.5 % and 2.5 % to 3 % by weight, calculated to the total hair dyeing composition (excluding the oxidation agent), whereby these figures are always related to the proportion of free base; the preferred weight proportion of the developing substances to the additional developing and coupling substances ranges between about 1 : 8 to 8 : 1, preferably about 1 : 5 to 5 : 1, in particular 1 : 2 to 2 : 1.

In the hair dyeing compositions according to the invention, the coupling substance(s) as reaction partners of the developing substance(s) are present in approximately the same molecular proportions as the developing substances, i.e. in amounts from 0.05 % to 5.0 %, preferably 0.1 % to 4 %, in particular 0.5 % to 3 % by weight, calculated to the total composition (excluding the oxidizing agent), whereby these figures are always related to the proportion of free base.

If desired, the compositions according to the invention can also contain so-called shading agents for precise adjustment of the desired shade, in particular direct-acting dyestuffs.

Such shading agents are, for example, nitro dyestuffs such as 2-amino-4,6-dinitrophenol, 2-amino-4-nitrophenol, 2-amino-6-chloro-4-nitrophenol, etc., preferably in amounts from about 0.05 % to 2.5 %, in particular 0.1 % to 1 % by weight of the dyestuff composition (excluding the oxidizing agent).

The hair dyeing compositions according to the invention can comprise the basic substances and additives customarily found in such compositions, conditioning agents, etc., known as state of the art and described, for example, in the monography of K. Schrader, "Grundlagen und Rezepturen der Kosmetika", 2nd Ed. (Hüthig Buch Verlag, Heidelberg, 1989), pp. 782 to 815. They can be prepared as solutions, creams, gels or also in the form of aerosol products; suitable carrier material compositions are known as state of the art.

For application, the oxidation dyestuff precursor is mixed with an oxidizing agent. The preferred oxidizing agent is hydrogen peroxide, for example in a concentration of 2 % to 6 % by weight.
However, the use of other peroxides such as urea peroxide and melamine peroxide is also possible.

The pH-value of the ready-to-use hair dyeing composition, i.e. after mixing with peroxide, can be in a slightly acidic range, i.e. from 5.5 to 6.9, as well as in the neutral or alkaline range, i.e. between pH 7.1 and 10.

In the following, various Examples are used to illustrate the invention.

### Carrier

| **Carrier** | |
|---|---|
| Stearyl alcohol | 8.0 (% by wt.) |
| Coco fatty acid monoethanolamide | 4.5 |
| 1,2-Propanediol mono/distearate | 1.3 |
| Coco fatty alcohol polyglycol ether | 4.0 |
| Sodium lauryl sulfate | 1.0 |
| Oleic acid | 2.0 |
| 1,2-Propanediol | 1.5 |
| Na-EDTA | 0.5 |
| Sodium sulfite | 1.0 |
| Protein hydrolyzate | 0.5 |
| Ascorbic acid | 0.2 |
| Perfume | 0.4 |
| Ammonia, 25% | 1.0 |
| Ammonium chloride | 0.5 |
| Panthenol | 0.8 |
| Water | ad 100.00 |

The oxidation dyestuff combinations according to the invention were incorporated into this carrier, whereby the water content was reduced accordingly.

The colorations were carried out on wool patches and strands of bleached human hair by application of a 1:1 mixture of a dyestuff precursor and a 6 % hydrogen peroxide solution (pH-value of the mixture: 9.8) with twenty minutes processing at room temperature, subsequent rinsing and drying.

The following colorations were achieved:

| **Example 1:** | | |
|---|---|---|
| 0.41 | (% by wt.) | 3-Chloro-p-aminophenol HCI |
| 0.14 | | 2-Methyl-5-aminophenol |
| 0.12 | | o-Aminophenol |

| Coloration: | | |
|---|---|---|
| | Vibrant orange. | |

| **Example 2** | | |
|---|---|---|
| 0.41 | (% by wt.) | 3-Chloro-p-aminophenol HCI |
| 0.25 | | 5-(2-hydroxyethyl)-4-amino-4-methoxy-2-methyl phenol |
| 0.12 | | o-Aminophenol |

| Coloration: | | |
|---|---|---|
| | Vibrant orange. | |

| **Example 3:** | | |
|---|---|---|
| 0.41 | (% by wt.) | 3-Chloro-p-aminophenol HCI |
| 0.25 | | 2-Methyl-5-hydroxyethyl aminophenol |
| 0.12 | | o-Aminophenol |

| Coloration: | | |
|---|---|---|
| | Vibrant orange. | |

| **Example 4:** | | |
|---|---|---|
| 0.20 | (% by wt.) | 3-Chloro-p-aminophenol HCI |
| 0.16 | | 2-Chloro-p-aminophenol |
| 0.14 | | 2-Methyl-5-aminophenol |
| 0.12 | | o-Aminophenol |

| Coloration: | | |
|---|---|---|
| | Intensive brick red. | |

| **Example 5:** | | |
|---|---|---|
| 0.41 | (% by wt.) | 3-Chloro-p-aminophenol HCI |
| 0.09 | | 2-Methyl-5-aminophenol |
| 0.08 | | o-Aminophenol |
| 0.09 | | 2-Methylresorcinol |

| Coloration: | | |
|---|---|---|
| | Vibrant copper-orange. | |

| **Example 6:** | | |
|---|---|---|
| 0.41 | (% by wt.) | 3-Chloro-p-aminophenol HCI |
| 0.09 | | 2-Methyl-5-aminophenol |
| 0.08 | | o-Aminophenol |
| 0.08 | | Resorcinol |

| Coloration: | | |
|---|---|---|
| | Vibrant coppery orange. | |

| **Example 7:** | | |
|---|---|---|
| 0.41 | (% by wt.) | 3-Chloro-p-aminophenol HCI |
| 0.09 | | 2-Methyl-5-aminophenol |
| 0.08 | | o-Aminophenol |
| 0.21 | | 1-Methoxy-2-amino-4-β-hydroxyethyl aminobenzene H₂SO₄ |

| Coloration: | | |
|---|---|---|
| | Intensively vibrant mahogany-red. | |

| **Example 8:** | | |
|---|---|---|
| 0.20 | (% by wt.) | 3-Chloro-p-aminophenol HCI |
| 0.14 | | 2-Methyl-5-aminophenol |
| 0.12 | | o-Aminophenol |
| 0.20 | | 1-Phenyl-3-methylpyrazole-5-one |

| Coloration: | | |
|---|---|---|
| | Vibrant, intensive fire red. | |

## Claims

1. Hair dyeing composition on the basis of an oxidation dyestuff precursor reacting with peroxide, comprising at least one developing and/or coupling substance selected from each group
a) 3-chloro-p-acninophenol and
b) 2-methyl-5-aminophenol, 2-methyl-5-hydroxyethyl aminophenol, 2-methyl-5-γ-hydroxypropyl aminophenol, 2-methyl-5-methyl aminophenol, 2-methyl-5-ethyl-aminophenol, 2-methoxy-5-aminophenol, 2-methyl-4-methoxy-5-aminophenol and/or 2-methyl-4-methoxy-5-hydroxyethyl aminophenol and
c) o-aminophenol and/or 4-chloro-2-aminophenol.

## Patentansprüche

1. Haarfärbemittel auf Basis eines mit Peroxid reagierenden Oxidationsfarbstoff-Vorprodukts, enthaltend mindestens eine Entwickler- und/oder Kupplersubstanz jeweils ausgewählt aus den Gruppen
a) 3-Chlor-p-aminophenol und,
b) 2-Methyl-5-aminophenol, 2-Methyl-5-hydroxyethylaminophenol, 2-Methyl-5-γ-hydroxypropylaminophenol, 2-Methyl-5-methylaminophenol, 2-Methyl-5-ethyl-aminophenol, 2-Methoxy-5-aminophenol, 2-Methyl-4-methoxy-5-aminophenol und/oder 2-Methyl-4-methoxy-5-hydroxyethylaminophenol und
c) o-Aminophenol und/oder 4-Chlor-2-aminophenol.

## Revendications

1. Composition colorante capillaire à base d'un précurseur de colorant d'oxydation réagissant avec un peroxyde, comprenant au moins une substance de développement et/ou de couplage choisie dans chaque groupe
a) le 3-chloro-p-aminophénol, et
b) le 2-méthyl-5-aminophénol, le 2-méthyl-5-hydroxyéthylaminophénol, le 2-méthyl-5-γ-hydroxypropyl-aminophénol, le 2-méthyl-5-méthylaminophénol, le 2-méthyl-5-éthylaminophénol, le 2-méthoxy-5-aminophénol, le 2-méthyl-4-méthoxy-5-aminophénol et/ou le 2-méthyl-4-méthoxy-5-hydroxyéthylaminophénol, et
c) l'o-aminophénol et/ou le 4-chloro-2-aminophénol.
